# EUROPÄISCHE PATENTSCHRIFT

(11) **EP 0 721 321 B1**
(45) Veröffentlichungstag und Bekanntmachung des Hinweises auf die Patenterteilung: **11.11.1998**
(21) Anmeldenummer: 94927624.0
(22) Anmeldetag: 19.09.1994
(51) Int. Cl.: A61K 7/13, A61K 7/135

(54) **WASSERSTOFFPEROXID-ZUBEREITUNGEN**
HYDROGEN PEROXIDE PREPARATIONS
PREPARATIONS D'EAU OXYGENEE

(30) Priorität: 30.09.1993 DE 4333370
(43) Veröffentlichungstag der Anmeldung: 17.07.1996
(73) Patentinhaber: Henkel Kommanditgesellschaft auf Aktien, 40191 Düsseldorf (DE)
(72) Erfinder: HÖFFKES, Horst, D-40595 Düsseldorf (DE); NEUHAUS, Winifried, D-40822 Mettmann (DE); NELLES, Karin, D-40789 Monheim (DE)
(74) Vertreter: Wilk, Hans-Christof, Dr.
(86) Internationale Anmeldenummer: EP9403118
(87) Internationale Veröffentlichungsnummer: WO9508978

(56) Entgegenhaltungen:
- EP-A- 0 287 773
- EP-A- 0 308 825
- WO-A-91/18584
- DE-A- 2 205 461

## Beschreibung

Die Erfindung betrifft wäßrige Zubereitungen von Wasserstoffperoxid, die sich besonders als Oxidationsmittelzubereitungen zum Bleichen und zur oxidativen Färbung der Haare mit Oxidationsfärbemitteln eignen.

Das Blondieren und Färben der Haare mit Oxidationshaarfärbemitteln wird in der Regel mit Hilfe von zwei separat verpackten Zubereitungen durchgeführt - der Blondiercreme oder Oxidationshaarfärbecreme (A) und der Oxidationsmittelzubereitung (B) - die erst kurz vor der Anwendung vereinigt und in einer Applikationsflasche durch einfaches Schütteln vermischt und dann als gebrauchsfertige Blondier- oder Färbezubereitung auf das Haar gebracht werden. Für die Heimanwendung wurden auch Zweikammer-Misch- und Abgabebehälter entwickelt, in welchen die Komponenten A und B im geeigneten Mengenverhältnis getrennt voneinander abgepackt sind und die eine von außen mechanisch zerstörbare Trennwand zwischen den beiden Kammern aufweisen sowie eine Abgabeöffnung an einer der beiden Kammern. Kurz vor der Anwendung wird die Trennwand zerstört, worauf die Komponenten (A) und (B) vereinigt und durch Schütteln oder andere mechanische Maßnahmen vermischt werden. Dann kann die gebrauchsfertige Haarfarbe- oder Blondierzubereitung durch die Abgabeöffnung entnommen bzw. direkt auf das zu färbende Haar aufgetragen werden.

Eine rasche und homogene Vermischung der Blondier- oder Färbecreme (A) und der Oxidationsmittelzubereitung (B) ist dabei für das Gelingen des Färbevorganges sehr wichtig. Man hat versucht, diese Vermischung z.B. dadurch zu erleichtern, daß man beide Komponenten bezüglich ihrer Zusammensetzung und ihrer Viskosität aufeinander abgestimmt hat, wie dies z.B. in DE 35 34 471 A1 oder in DE 37 32 147 vorgeschlagen wurde.

Es hat sich nun gezeigt, daß erhebliche Probleme nicht nur durch die unterschiedliche Zähigkeit der zu vermischenden Komponenten, sondern auch dadurch verursacht werden, daß es beim Vermischen zu starker Schaumbildung kommt. Durch die übermäßige Schaumentwicklung wird die mechanische Durchmischung der flüssigen Komponenten (A) und (B) erheblich behindert.

Die Schaumentwicklung wird dabei vor allem dadurch verursacht, daß die Oxidationsmittelzubereitungen, in der Regel wäßrige Zubereitungen von Wasserstoffperoxid, zur Stabilisierung stark schäumende anionische Tenside enthalten. Diese sind erforderlich, um die in solchen Zubereitungen zum Viskositätsaufbau nach dem Vermischen enthaltenen Dispersionen verdickender carboxyl- und/oder carboxylatgruppenhaltiger Polymerisate zu stabilisieren. Auch enthalten Blondier- und Oxidationsfärbemittelcremes häufig stark schäumende anionische Tenside, um die in diesen Produkten zum Viskositätsaufbau dispergierten Fettalkohole im dispergierten Zustand zu halten. Da man auf die Verwendung dieser stark schäumenden Aniontenside aus den genannten Gründen nicht verzichten kann, stellt sich das Problem, die Schaumentwicklung beim Vermischen der beiden flüssigen Komponenten (A) und (B) durch einen geeigneten Zusatz zu kontrollieren.

Darüber hinaus soll sich die durch Vermischen von (A) und (B) erhaltene Zubereitung nach erfolgter Anwendung auf dem Haar entgegen des beim Vermischen gewünschten Schäumverhaltens unter möglichst hoher Schaumentwicklung wieder mit Wasser aus dem Haar ausspülen lassen.

Aufgabe der Erfindung ist es daher, eine wäßrige, Aniontensid und carboxyl- und/oder carboxylatgruppenhaltiges Polymerisat enthaltende H₂O₂-Zubereitung (B) bereitzustellen, die beim Vermischen mit einer wasserstoffperoxidfreien, aniontensidhaltigen wäßrigen Blondier- und Färbecreme (A) wenig schäumt, sich nach der Anwendung auf dem Haar jedoch unter hoher Schaumentwicklung mit Wasser wieder ausspülen läßt.
Darüber hinaus soll die H₂O₂-Zubereitung über einige Monate hinweg bei 45°C lagerbeständig sein.

Gegenstand der Erfindung sind wäßrige Zubereitungen von Wasserstoffperoxid zum oxidativen Färben und Bleichen von Haaren mit einem pH-Wert von 2 bis 6 enthaltend ein anionisches Tensid, ein wasserlösliches oder wasserdispergierbares, verdickendes carboxyl- und/oder carboxylatgruppenhaltiges Polymerisat oder Copolymerisat, und gegebenenfalls mit bis zu 50 Ethylenoxid-Molekülen ethoxylierten C₁₂-C₁₈-Fettalkohol oder hydriertes Rizinusöl, dadurch gekennzeichnet, daß ein Schaumregulator, der aus Polysiloxan mit einem Gehalt an feinteiliger, hydrophobierter Kieselsäure besteht, in einer Menge von 0,001 bis 0,05 Gew.-%, vorzugsweise 0,003 bis 0,01 Gew.-%, bezogen auf die gesamte Zubereitung, enthalten ist, wobei der Gehalt an feinteiliger, hydrophobierter Kieselsäure 0,5 bis 20 Gew.-%, vorzugsweise 1 bis 15 Gew.-%, bezogen auf den Schaumregulator, beträgt.

Die erfindungsgemäßen Zubereitungen von Wasserstoffperoxid liegen bevorzugt als wäßrige Dispersion des carboxyl- und/oder carboxylatgruppenhaltigen Polymerisats- oder Copolymerisats vor, sie können aber darüber hinaus auch eine emulgierte bzw. dispergierte Fettphase enthalten. Der Gehalt an Wasserstoffperoxid liegt bevorzugt bei 1 - 12 Gew.-% der Zubereitung.

Als carboxyl- und/oder carboxylatgruppenhaltiges Polymerisat oder Copolymerisat wird bevorzugt ein Polymerisat oder Copolymerisat der Acrylsäure oder der Methacrylsäure eingesetzt. Solche Polymerisate und Copolymerisate sind an sich bekannt und werden als Verdickungsmittel für wäßrige Lösungen seit langem eingesetzt. In

GB-A 870 994 werden z.B. Dispersionen von Copolymerisaten aus wenigstens 10 Gew.-% Acrylsäure-Niedrigalkylester, 25 bis 70 Gew.-% Methacrylsäure und bis zu 40 Gew.-% eines weiteren Comonomeren mit einem Feststoffgehalt von 25 bis 50 Gew.-% beschrieben. Aus DE-A 11 64 095 sind Mischpolymerisate aus 50 - 75 Gew.-% Ethylacrylat, 25 - 35 Gew.-% Acrylsäure und 0 bis 25 Gew.-% anderer Comonomerer bekannt.

Diese Copolymerisate können durch Zugabe von vernetzend wirkenden, mehrfach ungesättigten copolymerisierbaren Comonomeren im Molekulargewicht und damit in der Verdickungswirkung modifiziert werden. Besonders wirksame Acrylatdispersionen für diesen Zweck sind z.B. aus DE-A 34 45 549 oder auch aus EP 0 398 576 A2 bekannt.

Die geeigneten Polymerisate stellen meist wäßrige Dispersionen, üblicherweise mit einem Feststoffgehalt von 20 bis 30 Gew.-% dar, die in einem pH-Bereich von etwa 2 bis 6 stabil und dünnflüssig sind. In dem Maße, wie der pH-Wert der verdünnten Lösungen durch wäßrige Basen, z.B. durch Alkalihydroxidlösungen (z.B. Natronlauge oder Kalilauge), Ammoniaklösung, Alkanolamine (z.B. Mono-, Di- oder Triethanolamin) angehoben und die Carboxylgruppen in die Salzform überführt werden, beginnen die Polymerketten sich zu entknäueln und in Lösung zu gehen, wobei sich die Viskosität der Lösung erhöht. Bei einem pH-Wert von ca. 8 ist die völlige Ionisation der Carboxylgruppen und die Viskositätsentwicklung abgeschlossen und klare Wasserlöslichkeit erreicht.

Die Polymerisate sind in den erfindungsgemäßen Zubereitungen bevorzugt in einer Menge von 0,1 - 10 Gew.-% (berechnet als Feststoff) bezogen auf die Zubereitung enthalten.

Zur Stabilisierung enthalten die erfindungsgemäßen Zubereitungen anionische Tenside. Geeignete anionische Tenside sind gekennzeichnet durch eine bevorzugt lineare Alkylgruppe oder Alkenylgruppe mit 12 bis 18 C-Atomen und eine daran gebundene anionische Gruppe, z.B. eine -SO₃(-) oder -O-(C₂H₄O)_{z}-SO₃⁽⁻⁾-Gruppe, in der z = 0 oder eine Zahl bis 20 sein kann.

Beispiele für solche bevorzugten Aniontenside sind Alkylsulfate, Alkansulfonate, α-Olefinsulfonate, Alkylpolyglycolethersulfate und Alkylpolyglycolethersulfonate. Weiterhin geeignete Aniontenside sind Sulfobernsteinsäure-monoester-Salze, Alkylpolyglucolethercarboxylate, Ölsäuresulfonate und andere schaumstarke, anionische Sulfat- oder Sulfonat-Tenside. Die anionischen Tenside liegen bevorzugt in Form ihrer Alkali-, Magnesium-, Ammonium-, oder Mono-, Di- oder Trialkanolammoniumsalze mit 2 - 4 C-Atomen in der Alkanolgruppe vor. Sie sind bevorzugt in einer Menge von 0,1 - 10 Gew.-% der Zubereitung enthalten.

Die zur Schaumregulierung erfindungsgemäß eingesetzten Schaumregulatoren sind in einer Menge von 0,001 bis 0,05 Gew.-%, vorzugsweise 0,003 bis 0,01 Gew.-%, bezogen auf die gesamte H₂O₂-Zubereitung, enthalten. Die Schaumregulatoren bestehen überwiegend aus Polysiloxan bzw. einem Gemisch von verschiedenen Polysiloxanen und 0,5 bis 20 Gew.-%, vorzugsweise 1 bis 15 Gew.-%, hydrophobierter Kieselsäure.

Unter feinteiliger, hydrophobierter Kieselsäure versteht man durch Behandeln von mikrofeiner, durch Fällung aus Silikatlösungen, durch Entwässerung von Kieselsäurehydrogel oder durch pyrogene Zersetzung von Siliciumtetrachlorid hergestellte Kieselsäure, die in bekannter Weise mit hyrophobierenden Reagenzien, z.B. Fettalkoholen, Fettaminen, Wachsen, insbesondere aber Organochlorsilanen umgesetzt wurde, wie dies z.B. in US 3,207,698 und US 3,388,073 beschrieben ist. Als Beispiel sei ein mit Dimethylchlorsilan oder mit Trimethylchlorsilan umgesetztes, pyrogenes Siliciumdioxid genannt. Weitere Beispiele sind die Handelsprodukte Kieselsäure-HDK-H 2000 der Fa. Wacker und Sipernat-D-10 oder Aerosil-R972 der Fa. Degussa.

Die nach der Methode von Brunauer, Emmett und Teller (BET) bestimmte spezifische Oberfläche der hydrophobierten Kieselsäuren liegt dabei zwischen 50 und 600 m²/g, vorzugsweise 110 bis 180 m²/g.

Das Polysiloxan hat eine Viskosität von 100 bis 60.000 mPa·s, vorzugsweise 1500 bis 3000 mPa·s, insbesondere 2000 bis 2500 mPa·s, gemessen mit einem Brookfield-Viskosimeter, Modell RVF, unter Verwendung von Spindel Nr. 5 bei 10 Umdrehungen pro Minute und 25°C.

Mögliche Polysiloxane sind z.B. Methylphenylpolysiloxane` aminomodifizierte Silikone, fettsäuremodifizierte Silikone, alkoholmodifizierte Silikone, polyethermodifizierte Silikone, epoxymodifizierte Silikone oder cyclische Silikone wie sie z.B. in EP 0 398 177 A2 offenbart werden.

Vorzugsweise wird jedoch Dimethylpolysiloxan eingesetzt. Beispiele sind z.B. Dow Corning 200 Fluid 1000; Abil 1000, Abil 1500, Abil 5000 der Firma Goldschmidt.

Die zur Schaumregulierung erfindungsgemäß eingesetzten Schaumregulatoren (Polysiloxan + hydrophobierte Kieselsäure) sind als solche im Handel erhältlich, z.B. unter dem Handelsnamen Dow Corning Compound DB-100.

Fakultativ können weitere übliche Schaumregulatoren wie z.B. Paraffine oder die in der deutschen Offenlegungsschrift DE 40 18 259 A1 offenbarten endgruppenverschlossenen Fettalkylpolyglykolether enthalten sein.

Als eine bevorzugte Ausführung der Erfindung ergibt sich eine wäßrige Zubereitung von Wasserstoffperoxid, enthaltend
- 1 - 12 Gew.-%: Wasserstoffperoxid
- 0,1 - 10 Gew.-%: anionisches Tensid
- 0,1 - 10 Gew.-%: eines verdickenden carboxyl- und/oder carboxylatgruppenhaltigen Polymerisats oder Copolymerisats und
- 0,001-0,05 Gew.-%,: vorzugsweise 0,003 bis 0,01 Gew-%, eines Schaumregulators, wobei der Gehalt an feinteiliger, hydrophobierter Kieselsäure 0,5 bis 20 Gew.-%, vorzugsweise 1 bis 15 Gew.-%, bezogen auf den Schaumregulator, beträgt.

Neben der gewünschten Schaumregulierung (wenig Schaum beim Vermischen mit einer wasserstoffperoxidfreien, aniontensidhaltigen wäßrigen Blondier- oder Färbecreme (A); viel Schaum beim anschließenden Ausspülen vom Haar mit Wasser) besitzen die erfindungsgemäßen Mittel eine überraschend hohe Lagerstabilität auch bei Temperaturen von 45°C.

Nie weiter oben ausgeführt, kann die erfindungsgemäße Wasserstoffperoxid-Zubereitung auch eine emulgierte oder dispergierte Fettphase zur Trübung und zur Einstellung einer erhöhten Viskosität und besseren Mischbarkeit enthalten. Als Fettkomponenten können z.B. Paraffine, Vaseline, Wachse, Hartfette, Fettsäuremono- und -diglyceride und vor allem lineare, gesättigte Fettalkohole, bevorzugt Cetyl- und Stearylalkohol in einer Menge von 0,5 - 5 Gew.-% der Zubereitung zugesetzt werden. Diese Fettkomponenten können entweder bereits in dispergierter Form zugemischt oder mit Hilfe des anionischen und nichtionischen Tensids in der Zubereitung dispergiert werden.

Zusätzlich zu den genannten Komponenten können die erfindungsgemäßen Wasserstoffperoxid-Zubereitungen noch weitere Hilfsmittel in untergeordneten Mengen enthalten. Solche Hilfsmittel sind z.B.
- Stabilisatoren für das Wasserstoffperoxid, wie z.B. Dipicolinsäure, Chinolinsäure, Polyphosphate oder die aus DE-B 11 07 207 bekannten Acylierungsprodukte der phosphorigen Säure, z.B. die 1-Hydroxyethan-1,1-diphosphonsäure in einer Menge von ca. 0,05 bis 1,5 Gew.-%
- Puffersubstanzen zur Einstellung eines pH-Wertes von 2 bis 6, bevorzugt saures Natriumpyrophosphat (Na₂H₂P₂O₇)
- wasserlösliche Proteinderivate, wasserlösliche kationische Polymere oder andere haarkosmetisch wirksame Komponenten
- und gegebenenfalls Duftstoffe.

Die zum Blondieren oder Färben der Haare verwendeten Blondier- oder Färbezubereitungen (A) stellen bevorzugt Öl-in-Wasser-Emulsionen (Cremes) dar. Als Fettkomponenten enthalten sie bevorzugt die bereits weiter oben genannten Fettkomponenten, bevorzugt Cetyl- und Stearylalkohol. Die Färbecremes dienen als Träger für die darin gelösten Oxidationsfarbstoffvorprodukte. Im Falle von Blondiermitteln enthalten diese Öl-in-Wasser-Emulsionen nur gelegentlich kleinere Mengen an Oxidationsfarbstoffvorprodrukten, die bei der Anwendung eine Kompensation zu starker, natürlicher Gelb- und Rotnuancen des Haares bewirken. Die Blondier- und Färbecremes werden bevorzugt auf einen pH-Wert von 8 - 11 und werden vor der Anwendung mit der erfindungsgemäßen Wasserstoffperoxid-Zubereitung vermischt.

Ein weiterer Gegenstand der Erfindung ist daher ein Verfahren zum Färben oder Aufhellen der Haare mit einer Oxidations-Haarfärbecreme oder Blondierungscreme (A) in Form einer Öl-in-Wasser-Emulsion mit einem pH-Wert von 6,5 - 11, indem man
die Oxidations-Haarfärbe- oder Blondierungscreme mit einer erfindungsgemäßen Wasserstoffperoxid-Zubereitung (B) mit einem pH-Wert von 2 - 6 in einem Gewichtsverhältnis (A):(B) = 3:1 bis 1:2 vermischt und
das dabei gebildete, gebrauchsfertige Haarfärbe- und Blondierungsmittel auf das Haar aufbringt und
nach einer Einwirkungszeit von 15 - 60 Minuten bei Raumtemperatur mit Wasser abspült.

Die Vermischung der erfindungsgemäßen Wasserstoffperoxidzubereitungen läßt sich durch leichtes Schütteln in der Applikationsflasche ohne großen Energieaufwand oder lange Schüttelzeiten durchführen. Die dabei erzielte Homogenität der Färbe- oder Blondierzubereitungen zeigt sich in einem verbesserten Färbeergebnis.

Ein weiterer Erfindungsgegenstand schließlich ist die Verwendung von Schaumregulatoren, die aus Polysiloxan mit einem 0,1 bis 20 Gew.-%igen, vorzugsweise 1 bis 15 Gew.-%igen, Gehalt an hydrophobierter Kieselsäure bestehen, zur Schaumregulierung in wäßrigen Zubereitungen, die Wasserstoffperoxid und Aniontensid enthalten.

Die folgenden Beispiele sollen den Erfindungsgegenstand näher erläutern:

### Beispiele

### 1. Wasserstoffperoxid-Zubereitungen

| | 1 | 2 | 3 |
|---|---|---|---|
| | Gew.% | Gew% | Gew% |
| Wasserstoffperoxid (50%ig in H₂O) | 12,0 | 12,0 | 12,0 |
| Lauryl-/Myristyl(70:30)(3EO)-ethersulfat, Na-Salz (28 gew.%ig in H₂O) | 2,0 | 2,0 | 2,0 |
| Ethylacrylat-Methacrylsäure-copolymerisat, 25 Gew% in H₂O (Latekoll^{(R)}D) | 15,0 | 15,0 | 15,0 |
| Kokosfettalkyl-(5EO)-n-butylether | - | 0,5 | - |
| Dimethylpolysiloxan, Viskosität 2000 bis 2500 mPa·s, enthaltend 10 - 20 Gew.-% hydrophobierte Kieselsäure (Dow Corning Compound DB-100) | 0,0067 | - | - |
| 1-Hydroxyethan-1,1-diphosphonsäure | 1,0 | 1,0 | 1,0 |
| Hydriertes, ethoxyliertes Rizinusöl 40 EO (Eumulgin HRE40 der Fa. Henkel) | 0,05 | 0,05 | 0,05 |
| Wasser | ad 100 | ad 100 | ad 100 |
| (EO : Ethylenoxid) | | | |

Zubereitung 1 ist erfindungsgemäß, die Zubereitungen 2 und 3 dienen zum Vergleich. 2 enthält als Schaumregulator einen endgruppenverschlossenen Fettalkylpolyglykolether, 3 enthält keinen Schaumregulator.

### Typische Oxidationshaarfärbecreme

| | |
|---|---|
| Fettalkoholgemisch C₁₂-C₁₈ | 11,0 Gew.% |
| Lauryl-/Myristyl(70:3)(3EO)-ethersulfat, Na-Salz (28 gew%ig in H₂O) | 25,0 Gew.% |
| p-Aminophenol-hydrochlorid | 0,5 Gew.-% |
| p-Toluylendiamin-sulfat | 0,38 Gew.-% |
| Resorcin | 0,25 Gew.-% |
| p-Amino-o-cresol | 0,33 Gew.-% |
| Natriumsulfit | 1,0 Gew.-% |
| Ammoniumsulfat | 0,7 Gew.-% |
| Duftstoff | 0,2 Gew.-% |
| Wasser (und NH₃ bis pH = 9,5) | ad 100 Gew.-% |

Je 50 ml der Wasserstoffperoxidzubereitungen 1, 2 und 3 wurden in flexible 100 ml-Polyethylen-Applikationsfläschchen gefüllt. Die Oxidationshaarfärbecreme wurde in 50 ml-Tuben gefüllt.

Zur Färbung der Haare wurde der Inhalt je einer Tube Oxidationshaarfärbecreme in je eines der Applikationsfläschchen mit der Wasserstoffperoxid-Zubereitung 1 bis 3 gedrückt. Nach Aufschrauben der Verschlußkappe wurde der Inhalt durch Schütteln von Hand gemischt. Die nach dem Mischen erhaltenen gebrauchsfertigen Färbezubereitungen wurden in üblicher Weise auf das Haar aufgebracht und nach der Einwirkungszeit mit Wasser ausgewaschen.

Die Beurteilung der Schaumbildung beim Mischen, der Mischbarkeit der beiden Komponenten und der Schaumbildung beim Auswaschen mit Wasser erfolgte nach einer Notenskala von 1 bis 5:

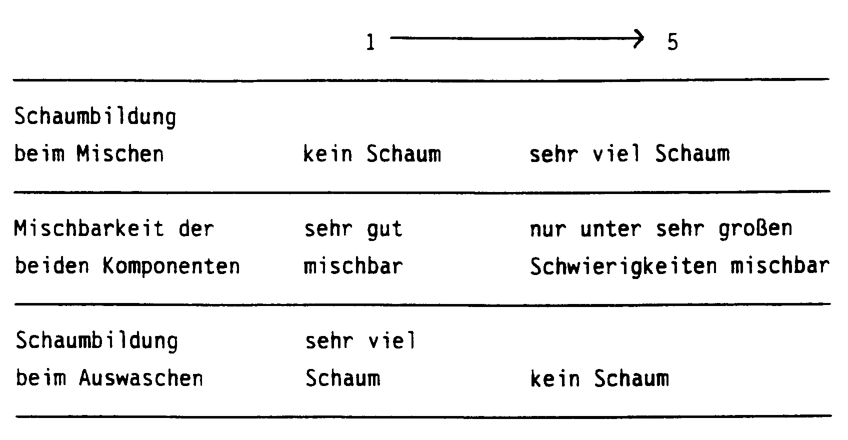

### Die Ergebnisse sind Tabelle 1 zu entnehmen:

**Tabelle 1**

| H₂O₂-Zubereitung 1, 2 oder 3 plus Färbecreme | Schaumbildung beim Mischen | Mischbarkeit der der beiden Komponenten | Schaumbildung beim Auswaschen |
|---|---|---|---|
| 1 | 2,0 | 2,0 | 2,0 |
| 2 | 2,0 | 2,0 | 2,0 |
| 3 | 4,0 | 4,0 | 1,5 |

Es zeigte sich, daß die nicht erfindungsgemäße Zubereitung 3 erst nach sehr langem Schütteln so homogen mit der Färbecreme gemischt war, daß mit bloßem Auge keine Inhomogenitäten mehr zu erkennen waren. Der Grund für die schlechtere Durchmischung lag offensichtlich in der starken Schaumentwicklung, durch welche die mechanische Bewegung der Flüssigkeit beim Schütteln behindert wurde.

Die erfindungsgemäße Zubereitung 1 und die nicht erfindungsgemäße Zubereitung 2 zeigten beim Vermischen mit der Färbecreme in etwa gleiches Schaumbildungs- und Mischungsverhalten.

Die Zubereitungen 1 und 2 wurden nun hinsichtlich eines weiteren für H₂O₂-Zubereitungen wichtigen Kriteriums, der Lagerstabilität, überprüft.
a) Die erfindungsgemäße Zubereitung 1 wurde 6 Monate lang bei 45°C gelagert (Abkürzung für diese Zubereitung: 1 GG)
b) Die Vergleichs-Zubereitung 2 wurde 4 Monate lang bei 20°C gelagert (Abkürzung für diese Zubereitung: 2 G)
c) Zum Vergleich wurde die Vergleichs-Zubereitung 2 frisch bereitet (Abkürzung für diese Zubereitung: 2 F)

Die Überprüfung der Lagerstabilität erfolgte auf die folgende Weise:

50 ml der H₂O₂-Zubereitung mit 50 ml der Färbecreme wurden in eine 120 ml-Applikationsflasche gefüllt. Die Höhe des Leervolumens von der Flüssigkeitsoberkante bis zur Schulter der Flasche betrug jeweils 24 mm.
Nach dem Einbringen der Färbecreme in die H₂O₂-Zubereitung wurde so intensiv geschüttelt, daß das gesamte Volumen oberhalb der Flüssigkeit mit dem Schaum erfüllt war. Nach Zeitabständen von 10 sec, 20 sec, 40 sec, 60 sec wurde dann die verbleibende Schaumhöhe bestimmt. Die Ergebnisse sind Tabelle 2 zu entnehmen:

**Tabelle 2**

| Zeit in Sekunden | verbleibende Schaumhöhe in mm | | |
|---|---|---|---|
| | 1 GG | 2 G | 2 F |
| 0 | 24 | 24 | 24 |
| 10 | 0 | 10 | 3 |
| 20 | 0 | 10 | 0 |
| 40 | 0 | 10 | 0 |
| 60 | 0 | 10 | 0 |

Es zeigte sich, daß die Schaumbildung beim Mischen bei der gelagerten erfindungsgemäßen Zubereitung 1 (1 GG) am geringsten war und das Schaumregulierungsvermögen selbst nach 6 Monaten Lagerzeit bei 45°C erhalten bleibt.

Die Überprüfung der Zubereitungen 1 GG und 2 G hinsichtlich der Mischbarkeit der beiden Komponenten (H₂O₂-Zubereitung + Färbecreme) und der Schaumbildung beim Auswaschen vom Haar führte zu folgenden Ergebnissen (Tabelle 3):

**Tabelle 3**

| H₂O₂-Zubereitung 1 GG oder 2 G plus Färbecreme | Schaumbildung beim Mischen | Mischbarkeit der beiden Komponenten | Schaumbildung beim Auswaschen |
|---|---|---|---|
| 1 GG | 2,0 | 2,0 | 2,0 |
| 2 G | 3,0 | 2,5 | 2,0 |

Auch diese Prüfung zeigte, daß die gelagerte erfindungsgemäße H₂O₂-Zubereitung 1 (1 GG) der unter schonenderen Bedingungen gelagerten nicht-erfindungsgemäßen H₂O₂-Zubereitung 2 (2 G) überlegen ist.

## Patentansprüche

1. Wäßrige Zubereitung von Wasserstoffperoxid zum oxidativen Färben und Bleichen von Haaren mit einem pH-Wert von 2 bis 6 enthaltend ein anionisches Tensid, ein wasserlösliches oder wasserdispergierbares, verdickendes carboxyl- und/oder carboxylatgruppenhaltiges Polymerisat oder Copolymerisat, und gegebenenfalls mit bis zu 50 Ethylenoxid-Molekülen ethoxylierten C₁₂-C₁₈-Fettalkohol oder hydriertes Rizinusöl, dadurch gekennzeichnet, daß ein Schaumregulator, der aus Polysiloxan mit einem Gehalt an feinteiliger, hydrophobierter Kieselsäure besteht, in einer Menge von 0,001 bis 0,05 Gew.-%, vorzugsweise 0,003 bis 0,01 Gew.-%, bezogen auf die gesamte Zubereitung, enthalten ist, wobei der Gehalt an feinteiliger, hydrophobierter Kieselsäure 0,5 bis 20 Gew.-%, vorzugsweise 1 bis 15 Gew.-%, bezogen auf den Schaumregulator, beträgt.

2. Zubereitung nach Anspruch 1, dadurch gekennzeichnet, daß das Polysiloxan eine Viskosität von 100 bis 60.000 mPa·s, vorzugsweise von 1500 bis 3000 mPa·s, gemessen mit einem Brookfield-Viskosimeter, Modell RVF, unter Verwendung von Spindel Nr. 5 bei 10 Umdrehungen pro Minute und 25°C aufweist.

3. Zubereitung nach Anspruch 1 und 2, dadurch gekennzeichnet, daß das Polysiloxan ein Dimethylpolysiloxan ist.

4. Zubereitung nach Anspruch 1 bis 3, dadurch gekennzeichnet, daß
1 - 12 Gew.-% Wasserstoffperoxid,
0,1 - 10 Gew.-% anionisches Tensid,
0,1 - 10 Gew.-% eines verdickenden carboxyl- und/oder carboxylatgruppenhaltigen Polymerisats oder Copolymerisats und
0,001 - 0,05 Gew.-%, vorzugsweise 0,003 bis 0,01 Gew.-%, eines Schaumregulators
enthalten sind, Mengenangaben jeweils bezogen auf die gesamte Zubereitung.

5. Verfahren zum Färben oder Aufhellen der Haare mit einer Oxidationshaarfärbecreme oder Blondierungscreme (A) in Form einer Öl-in-Wasser-Emulsion mit einem pH-Wert von 6,5 bis 11, dadurch gekennzeichnet, daß man die Haarfärbe- oder Blondierungscreme mit einer Wasserstoffperoxidzubereitung (B) nach einem der Ansprüche 1 bis 4 mit einem pH-Wert von 3 bis 5 in einem Gewichtsverhältnis von (A) : (B) gleich 3 : 1 bis 1 : 2 vermischt und das dabei gebildete gebrauchsfertige Haarfärbe- oder Blondierungsmittel auf das Haar aufbringt und nach einer Einwirkungszeit von 15 bis 60 Minuten bei Raumtemperatur mit Wasser abspült.

6. Verwendung von Schaumregulatoren, die aus Polysiloxan mit einem 0,1 bis 20 Gew.-%igen, vorzugsweise 1 bis 15 Gew.-%igen, Gehalt an hydrophobierter Kieselsäure bestehen, zur Schaumregulierung in wäßrigen Zubereitungen, die Wasserstoffperoxid und Aniontensid enthalten.

## Claims

1. A water-containing formulation of hydrogen peroxide for the oxidative dyeing and bleaching of hair with a pH value of 2 to 6 containing an anionic surfactant, a water-soluble or water-dispersible, thickening polymer or copolymer containing carboxyl and/or carboxylate groups and optionally C₁₂₋₁₈ fatty alcohol ethoxylated with up to 50 ethylene oxide molecules or hydrogenated castor oil, characterized in that they contain a foam regulator consisting of polysiloxane containing fine-particle hydrophobicized silica in a quantity of 0.001 to 0.05% by weight and preferably in a quantity of 0.003 to 0.01% by weight, based on the preparation as a whole, the content of fine-particle hydrophobicized silica being from 0.5 to 20% by weight and preferably from 1 to 15% by weight, based on the foam regulator.

2. A preparation as claimed in claim 1, characterized in that the polysiloxane has a viscosity in the range from 100 to 60,000 mPa·s and preferably in the range from 1,500 to 3,000 mPa·s, as measured with a Brookfield RVF viscosimeter, spindle No. 5, at 10 r.p.m./25°C.

3. A preparation as claimed in claims 1 and 2, characterized in that the polysiloxane is a dimethyl polysiloxane.

4. A preparation as claimed in claim 1, characterized in that it contains
1 to 12 % by weight of hydrogen peroxide,
0.1 to 10 % by weight of anionic surfactant,
0.1 to 10 % by weight of a thickening polymer or copolymer containing carboxyl and/or carboxylate groups and
0.001 to 0.05% by weight and preferably 0.003 to 0.01% by weight of a foam regulator,
based on the preparation as a whole.

5. A process for dyeing or lightening hair with an oxidation hair dyeing cream or bleaching cream (A) in the form of an oil-in-water emulsion with a pH value of 6.5 to 11, characterized in that the hair dyeing or bleaching cream is mixed with a hydrogen peroxide preparation (B) according to claims 1 to 4 with a pH value of 3 to 5 in a ratio by weight of (A) to (B) of 3:1 to 1:2, the ready-to-use hair dyeing and bleaching preparation formed is applied to the hair and is rinsed out with water after a contact time of 15 to 60 minutes at room temperature.

6. The use of foam regulators consisting of polysiloxane with a 0.1 to 20% by weight and preferably 1 to 15% by weight content of hydrophobicized silica for foam regulation in aqueous preparations containing hydrogen peroxide and anionic surfactant.

## Revendications

1. Préparation aqueuse d'eau oxygénée pour la coloration et la décoloration par oxydation des cheveux, possédant un pH de 2 à 6, contenant un tensioactif aniorique, un polymère ou un copolymère épaississant soluble ou dispersible dans l'eau, (enfermant des groupes carboxyle et/ou carboxylate et, le cas échéant, un alcool en C₁₂-C₁₈ éthoxylé avec jusqu'à 50 molécules d'oxyde d'éthylène, ou de l'huile de ricin hydrogénée caracterisée en ce qu'un régulateur de mousse, qui est constitué de polysiloxane renfermant de l'acide silicique rendu hydrophobe à fines particules, est contenu dans une proportion de 0,001 à 0,05 % en poids, de préférence de 0,003 à 0,01 % en poids, par rapport à la totalité de la préparation, la concentration en acide silicique rendu hydrophobe à fines particules atteignant de préférence 0,5 à 20 % en poids, de préférence 1 à 15 % en poids par rapport au régulateur de mousse.

2. Préparation selon la revendication 1, caractérisée en ce que le polysiloxane présente une viscosité de 100 à 60.000 mPa.s, de préférence de 1500 à 3000 mPa.s, déterminée avec un viscosimètre Brookfield, modèle RVF, en utilisant la broche n° 5 à 10 tours/minutes et à 25 °C.

3. Préparation selon la revendication 1 ou 2, caractérisée en ce que le polysiloxane est un diméthylpolysiloxane.

4. Préparation selon la revendication 1 à 3, caractérisée en ce qu'elle contient
1 à 12 % en poids d'eau oxygénée
0,1 à 10 % en poids de surfactif anionique
0,1 à 10 % en poids d'un polymère ou d'un copolymère épaississant, renfermant des groupes carboxyle et/ou carboxylate et
0,001 à 0,05 % en poids, de préférence 0,003 à 0,01 % en poids d'un régulateur de mousse,
les indications de quantités se rapportant dans chaque cas à la totalité de la préparation.

5. Procédé de coloration ou d'éclaircissement des cheveux par une crème capillaire colorante par oxydation ou décolorante (A), sous la forme d'une émulsion huile dans eau possédant un pH de 6,5 à 11, caractérisé en ce qu'on mélange la crème capillaire colorante ou décolorante avec une préparation d'eau oxygénée (B) selon une des revendications 1 à 4, présentant un pH de 3 à 5, dans un rapport pondéral (A):(B) de 3:1 à 1:2, on applique sur la chevelure le produit colorant ou décolorant pour les cheveux prêt à l'emploi ainsi formé et on le rince à l'eau à température ambiante après un temps d'action de 15 à 60 minutes.

6. Utilisation de régulateurs de mousse, qui sont constitués de polysiloxane contenant 0,1 à 20 % en poids, de préférence 1 à 15 % en poids d'acide silicique rendu hydrophobe, pour la régulation de la mousse dans les préparations aqueuses, qui contiennent de l'eau oxygénée et un tensioactif anionique.
